# EUROPEAN PATENT APPLICATION

(11) **EP 3 075 426 A1**
(43) Date of publication of application: **05.10.2016**
(21) Application number: 15831038.3
(22) Date of filing: 31.08.2015
(51) Int. Cl.: A63H 5/00

(54) **BLOW-OUT**

(30) Priority: 15.12.2014 JP 2014252847
(71) Applicant: Fujimura, Yoshio, Awaji-shi Hyogo 6562323 (JP); Yamamoto, Hirokazu, Miyoshi.shi Hiroshima 7280014 (JP); Kariya, Akemi, Mino-shi Osaka 5620024 (JP)
(72) Inventor: YAMAMOTO, Hirokazu, Miyoshi-shi Hiroshima 7280014 (JP); KARIYA, Akemi, Mino-shi Osaka 5620024 (JP); FUJIMURA, Yoshio, Awaji-shi Hyogo 6562323 (JP); KIMURA, Yoshikatsu, Hiroshima-shi Hiroshima 7340015 (JP)
(74) Representative: Williams, Michael Ian
(86) International application number: PCT/JP2015/074769
(87) International publication number: WO 2016/098396

(57) **Abstract**

[Problems] To provide an economical and handy blowout.

[Means for solving the Problems] A blowout includes: a stretchable body 2 having a tube shape and changeable in shape; a wire body 3 having elasticity and arranged along the longitudinal directions of the stretchable body 2; a pipe body 4 having a cylinder shape, one end part 41 of the pipe body 4 being connected to one end part 23 of the stretchable body 2; and a mouthpiece body 5 having a cylinder shape and connected to the other end part 42 of the pipe body 4, the mouthpiece body 5 being held by a user in the mouth such that air is blown by the user, in which the stretchable body 2 is stretched by blowing air from the mouthpiece body 5 while the stretchable body 2 is curled into a spiral by the elastic force of the wire body 3 when air is not blown from the mouthpiece body 5. In the blowout, the one end part 23 of the stretchable body 2 and the one end part 41 of the pipe body 4 are connected by a tightening band 7 so that the stretchable body 2 can be attached to and detached from the pipe body 4.

## Description

### [Technical Field]

The present invention relates to a blowout, particularly, an economical blowout.

### [Background Art]

A blowout has been widely known as a "fukimodoshi" toy since a long time ago, which includes a stretchable body (pouch-like portion) having a tube shape, and a wire spring arranged along the longitudinal directions of the stretchable body. If air is blown into the stretchable body from an end thereof, then the stretchable body is stretched, while the stretchable body is curled into a spiral by the elastic force of the wire spring when air is not blown into it.

Conventionally, the blowout is mainly utilized as a toy for children, but a blowout utilizable for health promotion, medical treatment, cosmetic treatment and the like is also known (e.g., refer to Patent Document 1). This type of blowout has a mouthpiece arranged at the air-blowing part thereof, the mouthpiece being formed of an antibacterial, flexible material and being attachable to and detachable from the blowout, so that the blowout can be kept hygienic even if it is repeatedly utilized over a long period of time.

### [Prior Art Documents]

### [Patent Document]

[Patent Document 1] Registered Utility Model Publication No. 3137944

### [Summary of the Invention]

### [Problems to be solved by the Invention]

However, the stretchable body is generally formed by a sheet of paper and may be degraded after the blowout is repeatedly utilized. Specifically, the stretchable body may not be stretched straight or not be curled into a spiral.

Further, the stretchable body is united by means of tape or the like to another portion (such as a pipe body). If the stretchable body is degraded, then the entire blowout needs disposing of, even though another such portion is not degraded, which is uneconomical. Still further, for the purpose of health promotion, cosmetic treatment or the like, a user has to keep utilizing a blowout over a long period of time. Hence, every time the stretchable body is degraded, the user has to replace the blowout itself and thereby bear a heavy economic burden.

In addition, a blowout is blown to stretch a stretchable body thereof, and this blowing force (required exhaling force) depends mainly upon the elastic force of a spring of the stretchable body. This requires that the stretchable body should be provided with a spring having an elastic force suitable for a usage purpose such as health promotion and cosmetic treatment. Similarly, in accordance with the usage purposes such as health promotion and cosmetic treatment, the stretchable body needs to have a suitable length such that the stretchable body will not be stretched over the full length unless a suitable quantity of air is blown therein.

However, the stretchable body is united to another portion, and hence, if a user wants to utilize a blowout which differs in required exhaling force (elastic force) or required quantity of air, then the user has to purchase an entire blowout anew and bear a heavy economic burden, thereby making the blowout unhandy.

Therefore, it is an object of the present invention to provide an economical and handy blowout.

### [Means for solving the Problem]

In order to solve the problems, a blowout according to claim 1 includes: a stretchable body having a tube shape and changeable in shape; a wire body having elasticity and arranged along the longitudinal directions of the stretchable body; a pipe body having a cylinder shape, one end part of the pipe body being connected to one end part of the stretchable body; and a mouthpiece body having a cylinder shape and connected to the other end part of the pipe body, the mouthpiece body being held by a user in the mouth such that air is blown by the user, in which the stretchable body is stretched by blowing air from the mouthpiece body while the stretchable body is curled into a spiral by the elastic force of the wire body when air is not blown from the mouthpiece body, wherein the one end part of the stretchable body and the one end part of the pipe body are connected by an attaching and detaching means so that the stretchable body is attachable to and detachable from the pipe body.

According to claim 2, in the blowout of claim 1: the attaching and detaching means is a tightening band having a ring shape and flexible in internal shape; and the one end part of the pipe body is inserted into the one end part of the stretchable body, the tightening band is tightened from above on the one end part of the stretchable body to connect the stretchable body and the pipe body, and the tightening band is loosened to detach the stretchable body from the pipe body.

According to claim 3, in the blowout of claim 1: the attaching and detaching means is an elastic body having flexibility, the elastic body being arranged in the one end part of the stretchable body such that the elastic body extends in the circumferential directions of the stretchable body; and the one end part of the pipe body is inserted into the one end part of the stretchable body and thereby is pressed by the elastic body to connect the stretchable body and the pipe body, and the pipe body is drawn from the stretchable body to detach the stretchable body from the pipe body.

According to claim 4, in the blowout of claim 1: the attaching and detaching means is constituted by a first thread engagement portion provided in the one end part of the stretchable body and a second thread engagement portion provided in the one end part of the pipe body, the first thread engagement portion being operable to engage with the second thread engagement portion; and the first thread engagement portion engages with the second thread engagement portion to connect the stretchable body and the pipe body, and the first thread engagement portion disengages from the second thread engagement portion to detach the stretchable body from the pipe body.

### [Advantages of the Invention]

In the blowout of claim 1, the stretchable body is attachable to and detachable from the pipe body. Therefore, even if the blowout is utilized and the stretchable body is degraded, then without disposing of the entire blowout, a new stretchable body can be attached to the pipe body. In short, only the stretchable body may be replaced and the pipe body, the mouthpiece body and the like can be continuously utilized, thereby making the blowout economical. Further, if a user wants to change the required exhaling force or the required quantity of air in accordance with the usage purposes or the like, then the user can attach, to the pipe body, a stretchable body having a desired/specified required exhaling force (elastic force) or required quantity of air. The user does not have to replace the entire blowout, and hence, the user may only purchase a desired stretchable body and attach the stretchable body, thereby lightening the economic burden and making the blowout handy.

In the blowout of claim 2, the tightening band is simply tightened or loosened, and thereby, the stretchable body can be securely, easily and quickly attached to or detached from the pipe body. Besides, the attaching and detaching means is formed by only the tightening band which is a separate body from the stretchable body or the pipe body. Therefore, the conventional stretchable body or pipe body can be directly utilized, thereby reducing the manufacturing cost and hence making the blowout more economical.

In the blowout of claim 3, the pipe body is simply inserted into the stretchable body (elastic body) or drawn from the stretchable body, and thereby, the stretchable body can be securely, easily and quickly attached to or detached from the pipe body.

In the blowout of claim 4, the first thread engagement portion simply engages with or disengages from the second thread engagement portion, and thereby, the stretchable body can be securely, easily and quickly attached to or detached from the pipe body.

### [Brief Description of the Drawings]

Fig. 1 is a perspective view of a blowout according to a first embodiment of the present invention.
Fig. 2 is an exploded perspective view of the blowout of the Fig. 1.
Fig. 3 is a plan view showing a stretchable body of the blowout of Fig. 1 being stretched.
Fig. 4 is a cross-sectional view showing how to arrange wire bodies of the blowout of Fig. 1.
Fig. 5 is a longitudinal sectional view of a mouthpiece body of the blowout of Fig. 1.
Fig. 6 is a perspective view of a tightening band of the blowout of Fig. 1.
Fig. 7 is an exploded perspective view of a blowout according to a second embodiment of the present invention.
Fig. 8 is an exploded perspective view of a blowout according to a third embodiment of the present invention.

### [Mode for implementing the Invention]

Embodiments of the present invention will be below described with reference to the drawings.

### (First Embodiment)

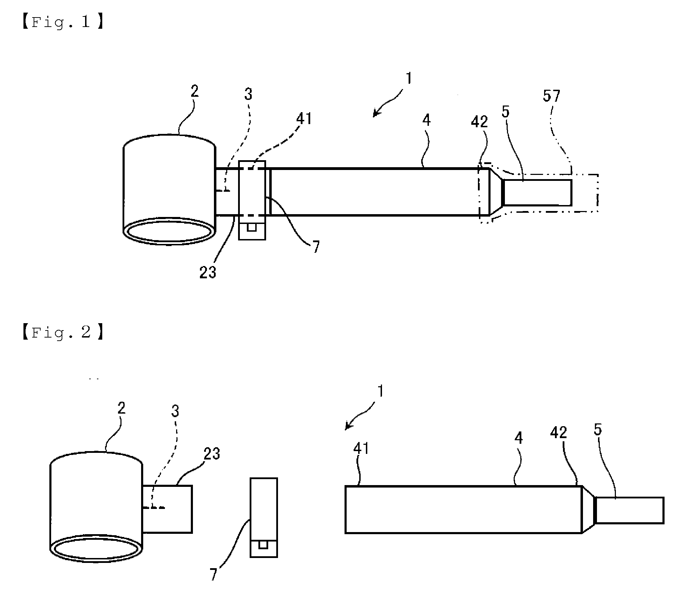

Figs. 1 to 6 show a first embodiment of the present invention, and Fig. 1 is a perspective view of a blowout 1 according to this embodiment and Fig. 2 is an exploded perspective view of the blowout 1. The blowout 1 is particularly utilizable for health promotion, medical treatment, cosmetic treatment and the like, and mainly includes a stretchable body 2, a wire body 3, a pipe body 4, a mouthpiece body 5, an air regulating body (flow-rate regulating means) 6 and a tightening band (attaching and detaching means) 7.

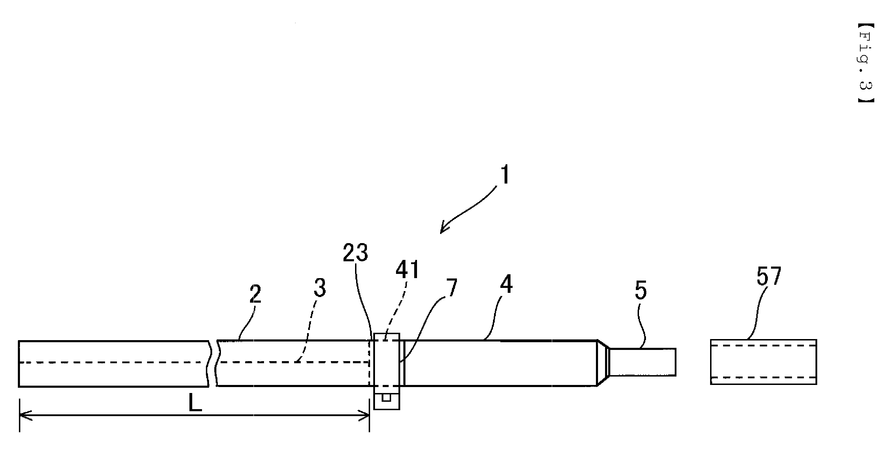

The stretchable body 2 is a pouch-like portion having a tube shape and changeable in shape, and is formed by sheets of paper 21 and 22 having predetermined material qualities (properties) such as thickness, strength, density (weight per unit area) and durability. As shown in Fig. 3, the stretchable body 2 has a circle shape in cross section when inflated with air blown into it, and the inner diameter thereof is designed to be slightly larger than the outer diameter of the pipe body 4. The stretchable body 2 is formed into a tube by bending a first material 21 having the shape of a substantially rectangular (substantially quadrilateral) sheet (described later).

A length L of the stretchable body 2 (except the insertion part of the pipe body 4 into the stretchable body 2) is set in accordance with the purposes (what the blowout 1 is used for) such as health promotion, medical treatment and cosmetic treatment, the user conditions such as the oral function condition and the physical condition of a user, or the like. Specifically, the length L of the stretchable body 2 is set such that the quantity of air necessary for stretching out the stretchable body 2 over the full length L equates with a suitable quantity of air preset for a purpose, a user condition or the like.

The wire body 3 is a wire having elasticity and arranged straight along the longitudinal directions of the stretchable body 2. Specifically, the wire body 3 is formed of stainless-steel wire and is curled into a spiral/coil when not given an external force (when air is not blown into the stretchable body 2). If the wire body 3 is released after straightened by an external force, the wire body 3 returns to the spiral shape by an elastic force of its own. The elastic force (elastic modulus) is set to a predetermined value such that the required exhaling force for stretching the stretchable body 2 becomes a suitable value set in accordance with the purposes, the user conditions or the like, in other words, such that the stretchable body 2 will not be stretched unless the force (exhaled air pressure) by which air is blown from the mouthpiece body 5 is equal to or more than the suitable value (predetermined value).

As the suitable value, for example, for the purpose of health promotion, different suitable values, in order from low, level 0, level 1 and level 2 can be set in accordance with the oral function condition, physical condition (age) or the like of a user. In short, suitable values for the blowing force (exhaled air pressure) are finely set in accordance with the purposes, the user conditions or the like.

Further, a plurality of the wire bodies 3 may be provided when the single wire body 3 cannot precisely offer the elastic force equivalent to a predetermined value, or when the plurality of wire bodies 3 can more precisely and easily offer the elastic force equivalent to a predetermined value.

The plurality of wire bodies 3 could ensure that the stretchable body 2 will not be stretched unless the force by which air is blown from the mouthpiece body 5 is equal to or more than a suitable value (predetermined value). For example, one wire body 3 offering the elastic force of a predetermined value is provided for the level 1, and two such wire bodies 3 each offering the elastic force of the same predetermined value are provided for the level 2.

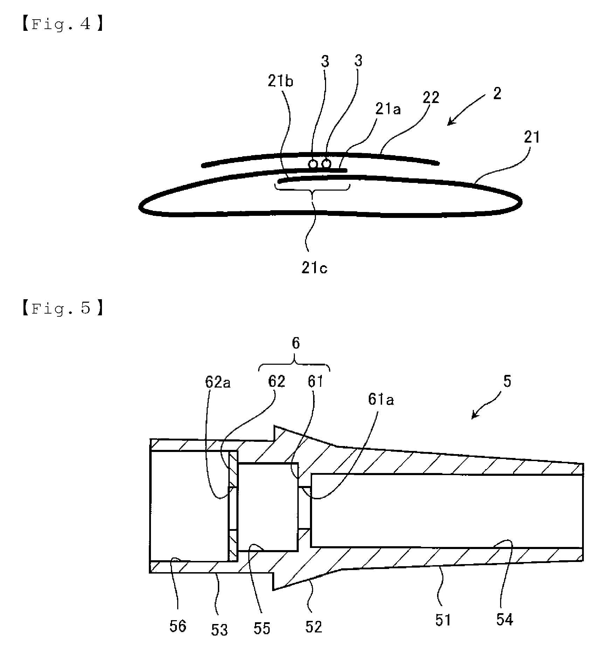

The wire body 3 is, as shown in Fig. 4, housed in the stretchable body 2 such that it is located inside of each ring of the spiral of the stretchable body 2. Specifically, the stretchable body 2 is formed by the substantially rectangular first material 21 and the long-strip shaped second material 22. The first material 21 is shaped into a tube by placing one end part 21a of the first material 21 on top of the other end part 21b of the first material 21 and gluing the one end part 21a to the other end part 21b. Then, the wire body 3 is arranged on the external surface (outer surface) of an overlap part 21c between the one end part 21a and the other end part 21b. In this state, the second material 22 is glued/joined to the first material 21 such that the second material 22 covers the whole external surface of the overlap part 21c, and thereby, the wire body 3 is united to the stretchable body 2.

Hence, the wire body 3 is arranged on the overlap part 21c between the one end part 21a and the other end part 21b of the first material 21, and then, the second material 22 is arranged on the wire body 3. Therefore, the wire body 3 is strongly held between the two materials 21 and 22, and thereby, is securely united to the stretchable body 2. Fig. 4 shows that the two wire bodies 3 are provided, and the number of the wire bodies 3 is set as previously described.

The pipe body 4 is a pipe portion which has a cylinder shape and one end part 41 of which is connected to one end part 23 of the stretchable body 2. Specifically, the pipe body 4 is a cylindrical body formed by a piece of thick paper having a specified quality of material, and as shown in Figs. 1 and 2, the one end part 41 is inserted into the one end part 23 of the stretchable body 2 and connected thereto with the tightening band 7 so that the pipe body 4 can be attached to and detached from the stretchable body 2 (as described later). In short, the one end part 23 of the stretchable body 2 and the one end part 41 of the pipe body 4 are connected by the tightening band 7 so that the stretchable body 2 can be attached to and detached from the pipe body 4. The pipe body 4 has such an inner diameter and a length that a user can hold it easily and blow air easily from the mouthpiece body 5 into the stretchable body 2.

The mouthpiece body 5 is a mouthpiece portion having a cylinder shape and connected to the other end part (the end part on the opposite side to the stretchable body 2) 42 of the pipe body 4. A user holds the mouthpiece body 5 in the mouth and blows air into the mouthpiece body 5. The mouthpiece body 5 is provided inside with the air regulating body 6 regulating the quantity of air flowing toward the stretchable body 2.

The mouthpiece body 5 is made of resin, and as shown in Fig. 5, includes a first barrel portion 51, a second barrel portion 52 and a third barrel portion 53. The first barrel portion 51 has a cylinder shape slightly tapering to one end thereof. The second barrel portion 52 is arranged at the other end of the first barrel portion 51 and has a cone shape small in outer diameter on the side of the first barrel portion 51. The third barrel portion 53 is arranged at the end of the second barrel portion 52 on the opposite side to the first barrel portion 51, and has an outer diameter substantially equal to the inner diameter of the pipe body 4. The third barrel portion 53 is inserted/pressed into the pipe body 4, and thereby, the mouthpiece body 5 is connected to the pipe body 4.

The mouthpiece body 5 is formed inside with: a first hole portion 54 located in the first barrel portion 51 and the second barrel portion 52; a second hole portion 55 located in the second barrel portion 52 and the third barrel portion 53; and a third hole portion 56 located in the third barrel portion 53. The hole portions 54-56 are concentric with the mouthpiece body 5 and have an inner diameter different from each other. The inner diameter becomes larger in the order of the first hole portion 54, the second hole portion 55 and the third hole portion 56 so that air easily flows toward the stretchable body 2.

The air regulating body 6 includes a restricting portion 61 united with the mouthpiece body 5 and restricting the flow rate of air, and a restricting body 62 having a ring shape, attached to the mouthpiece body 5 and restricting the flowrate of air. The restrictingportion 61 is apartition wall between the first hole portion 54 and the second hole portion 55, and is formed at the center with a first restriction hole 61a. From the first hole portion 54, air flows through the first restriction hole 61a and thereby the flow rate of the air is restricted, so that the air can be sent at a predetermined flow rate to the second hole portion 55.

The restricting body 62 is a metal disk having an outer diameter substantially equal to the inner diameter of the third hole portion 56, and is inserted/pressed into the third hole portion 56 and attached to the step part between the second hole portion 55 and the third hole portion 56. The restricting body 62 is formed at the center with a second restriction hole 62a. From the second hole portion 55, air flows through the second restriction hole 62a and thereby the flow rate of the air is restricted, so that the air is sent at a predetermined flow rate to the third hole portion 56. In this embodiment, the first restriction hole 61a and the second restriction hole 62a each have a diameter which is approximately half the diameter of the first hole portion 54.

As described above, the restricting portion 61 and the restricting body 62 restrict the flow rate of air blown into them and send the air to the stretchable body 2. Therefore, the stretchable body 2 will not be stretched unless the force by which air is blown from the mouthpiece body 5 is equal to or more than a predetermined value. In other words, the flow-rate restrictions (the diameters of the restriction holes 61a and 62a) of the restricting portion 61 and the restricting body 62 are set such that the stretchable body 2 will not be stretched unless the force (exhaled air pressure) by which air is blown from the mouthpiece body 5 is equal to or more than a suitable value set in accordance with the purposes, the user conditions or the like.

In consideration of the quality of material, inner diameter, length and the like of the stretchable body 2 and the pipe body 4, therefore, an elastic force (elastic modulus) of the wire body 3, the number of wire bodies 3 and a flow-rate restriction (regulated air quantity) by the air regulating body 6 are set such that the required exhaling force for stretching the stretchable body 2 becomes a suitable value set in accordance with the purposes, the user conditions or the like. In other words, the elastic force of the wire body 3 or the flow-rate restriction by the air regulating body 6 is adjusted such that the stretchable body 2 will not be stretched unless the force (exhaled air pressure) by which air is blown from the mouthpiece body 5 is equal to or more than a suitable value (predetermined value).

The mouthpiece body 5 is provided with a mouthpiece 57 attachable thereto. The mouthpiece 57 is formed of a flexible material (e.g., silicone) subjected to antibacterial treatment. As shown in Fig. 1, the mouthpiece 57 is a semitransparent cylinder, and into the cylinder, the mouthpiece body 5 is inserted, and then, is pressed into the pipe body 4 so that the mouthpiece 57 can be attached to the mouthpiece body 5. The mouthpiece 57 enables a user to hold the mouthpiece body 5 comfortably in the mouth and keeps the mouthpiece body 5 hygienic even if repeatedly utilized over a long period of time.

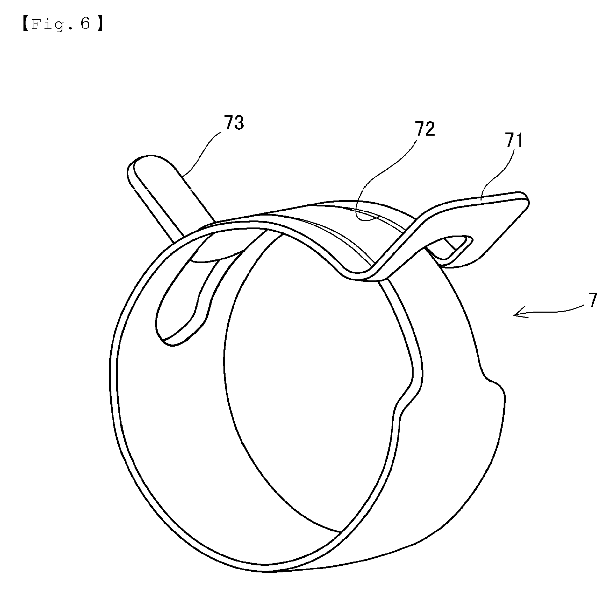

The tightening band 7 connects the one end part 23 of the stretchable body 2 and the one end part 41 of the pipe body 4 so that the stretchable body 2 can be attached to and detached from the pipe body 4. The tightening band 7 has a ring shape and is flexible in internal shape. As shown in Fig. 6, the tightening band 7 is formed by a long-strip shaped sheet (spring) having elasticity. In one end part 71 thereof, a long hole 72 is formed along the longitudinal directions, and the other end part 73 thereof is narrower than the one end part 71. The tightening band 7 is shaped into a ring (circle) by inserting/setting the other end part 73 into the long hole 72, and then, both end parts 71 and 73 are bent and protruded outward. Both end parts 71 and 73 are pinched to widen the inner diameter (internal shape) of the tightening band 7 so that the tightening band 7 is loosened. Then, both end parts 71 and 73 are released to narrow the inner diameter so that the tightening band 7 is tightened.

As shown in Fig. 1, the one end part 41 of the pipe body 4 is inserted into the one end part 23 of the stretchable body 2, and then, the tightening band 7 is tightened from above on the one end part 23 of the stretchable body 2 to connect the stretchable body 2 and the pipe body 4. On the other hand, the tightening band 7 is loosened to detach the stretchable body 2 from the pipe body 4. In the attachment and detachment, the tightening band 7 is set on the middle part of the pipe body 4, and then, the tightening band 7 is moved to the one end part 41 to connect the stretchable body 2 and the pipe body 4 while it is moved (apart) from the one end part 41 to detach the stretchable body 2 from the pipe body 4.

In the thus configured blowout 1, a user blows air from the mouthpiece body 5 (the mouthpiece 57), and thereby, as shown in Fig. 3, the air is sent into the stretchable body 2 to stretch the stretchable body 2. On the other hand, when air is not blown from the mouthpiece body 5, as shown in Fig. 1, the stretchable body 2 is curled into a spiral again by the elastic force of the wire body 3. In this process, the stretchable body 2 will not continue stretching unless the force (exhaled air pressure) by which air is blown from the mouthpiece body 5 is equal to or more than a suitable value (predetermined value) set in accordance with the purposes, the user conditions or the like. Unless the quantity of air blown from the mouthpiece body 5 reaches a preset quantity of air in accordance with the purposes, the user conditions or the like, the stretchable body 2 will not stretch out over the full length L. Therefore, a user utilizes the blowout 1 and stretches (blows out) the stretchable body 2 repeatedly, and thereby, the user can take exercise and do training with a blowing force suitable for a purpose, a user condition or the like.

As described above, in the blowout 1, the stretchable body 2 is attachable to and detachable from the pipe body 4. Therefore, even if the blowout 1 is utilized and the stretchable body 2 is degraded, then without disposing of the entire blowout 1, a new stretchable body 2 can be attached to the pipe body 4. In short, only the stretchable body 2 may be replaced, while the pipe body 4, the mouthpiece body 5 and the like can be continuously utilized, thereby making the blowout 1 economical.

In addition, the tightening band 7 is simply tightened or loosened, and thereby, the stretchable body 2 can be securely, easily and quickly attached to or detached from the pipe body 4. Besides, the attaching and detaching means is formed by only the tightening band 7 which is a separate body from the stretchable body 2 or the pipe body 4. Therefore, the conventional stretchable body 2 or pipe body 4 can be directly utilized (without an improvement/modification in the stretchable body 2 or the pipe body 4), thereby reducing the manufacturing cost and hence making the blowout 1 more economical.

Furthermore, if a user wants to change the required exhaling force or the required quantity of air in accordance with the usage purposes or the like, then the user can attach, to the pipe body 4, a stretchable body 2 having a desired/specified required exhaling force (elastic force) or required quantity of air. The user does not have to replace the entire blowout 1, and hence, the user may only purchase a desired stretchable body 2 and attach the stretchable body 2, thereby lightening the economic burden and making the blowout 1 handy. For example, a user may first attach a stretchable body 2 having a low-level required exhaling force (elastic force) to the pipe body 4 and utilize the stretchable body 2. Then, step by step after having become accustomed to it, the user can replace it with a stretchable body 2 having a high-level required exhaling force. This enables the user to take exercise and do training with a blowing force suitable for a purpose, a user condition or the like.

In particular, the elastic force of the wire body 3 is set to a predetermined value, and further, the air regulating body 6 is provided for regulating the quantity of air flowing toward the stretchable body 2. Therefore, the stretchable body 2 will not be stretched unless the force (exhaled air pressure) by which air is blown from the mouthpiece body 5 is equal to or more than a predetermined value. In other words, the required exhaling force (required exhaled-air pressure) for stretching the stretchable body 2 is set using both the elastic force of the wire body 3 and the regulation of the quantity of air (restrictions on the flow rate of air) by the air regulating body 6.

Accordingly, an optional/specified wire body 3 (stretchable body 2) and air regulating body 6 (pipe body 4) are combined so that the required exhaling force can be precisely adjusted and set to a suitable value for a purpose, a user condition or the like. Specifically, even if the blowing force (exhaled air pressure) needs to be finely adjusted in accordance with the purposes such as health promotion, medical treatment and cosmetic treatment, the user conditions such as the oral function condition and the physical condition of a user, or the like, then the specified stretchable body 2 and pipe body 4 are connected so that the required exhaling force can be optionally and precisely adjusted and varied.

Moreover, the air regulating body 6 includes the restricting portion 61 and the restricting body 62. Therefore, the flow-rate restrictions of both the restricting portion 61 and the restricting body 62 are adjusted, so that the quantity of air flowing toward the stretchable body 2 can be finely regulated more precisely. Accordingly, the required exhaling force for stretching the stretchable body 2 can be more precisely set to a suitable value for a purpose, a user condition or the like. Besides, the restricting body 62 is a separate body from the mouthpiece body 5, and hence, the restricting body 62 provided with the second restriction hole 62a having a different diameter can be attached to the mouthpiece body 5. Consequently, the flow-rate restriction (regulated air quantity) by the air regulating body 6 can be adjusted and varied easily without exchanging the mouthpiece body 5.

In addition, the wire body 3 is strongly held between the two materials 21 and 22 and is securely united to the stretchable body 2, and thereby, the elastic force of the wire body 3 is directly transmitted to the stretchable body 2. This means that the elastic force set for the wire body 3 directly affects the required exhaling force for stretching the stretchable body 2. Accordingly, simply by setting the elastic force of the wire body 3 to a predetermined value, the required exhaling force can be more precisely set to a desired suitable value.

If the wire body 3 is merely arranged along the stretchable body 2 and glued thereto, then the wire body 3 becomes a separate body from the stretchable body 2, thereby hindering the elastic force of the wire body 3 from being directly transmitted to the stretchable body 2. Consequently, the elastic force (recoiling force) of the stretchable body 2 differs from the elastic force of the wire body 3, thereby affecting and varying the required exhaling force for stretching the stretchable body 2. In contrast, the blowout 1 does not undergo the influence and variation because the wire body 3 is securely united to the stretchable body 2. Accordingly, the required exhaling force can be more precisely set to a desired suitable value.

Furthermore, the plurality of wire bodies 3 are provided, and the wire bodies adjust and determine the required exhaling force for stretching the stretchable body 2. Accordingly, the required exhaling force can be more precisely set to a desired suitable value.

### (Second Embodiment)

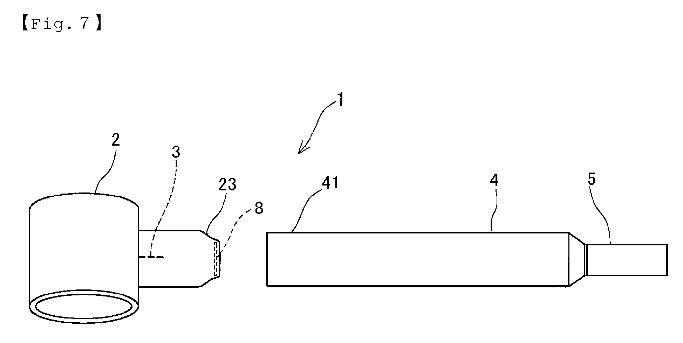

Fig. 7 is an exploded perspective view of a blowout 1 according to a second embodiment of the present invention. This embodiment is different from the first embodiment in the respect that the attaching and detaching means is formed by a rubber ring (elastic body) 8. The other component elements of the second embodiment are given the same reference numerals as those of the first embodiment, as long as the former are identical to the latter. Thus, their description is omitted.

The rubber ring 8 is an elastic body having flexibility and is arranged in the one end part 23 of the stretchable body 2 such that the rubber ring 8 extends in the circumferential directions of the stretchable body 2. Specifically, the rubber ring 8 is a rubber body having a ring shape and is arranged along the opening of the one end part 23 of the stretchable body 2. The elastic force and width thereof are designed so that the stretchable body 2 can be connected securely (without an air leak) to the pipe body 4 and so that a person can easily attach and detach the stretchable body 2 to and from the pipe body 4. The one end part 41 of the pipe body 4 is inserted into the one end part 23 of the stretchable body 2 and thereby is pressed by the rubber ring 8 to connect the stretchable body 2 and the pipe body 4. On the other hand, the pipe body 4 is drawn from the stretchable body 2 to detach the stretchable body 2 from the pipe body 4.

In the blowout 1 of this embodiment, the pipe body 4 is simply inserted into the stretchable body 2 (rubber ring 8) or drawn from the stretchable body 2, and thereby, the stretchable body 2 can be securely, easily and quickly attached to or detached from the pipe body 4.

### (Third Embodiment)

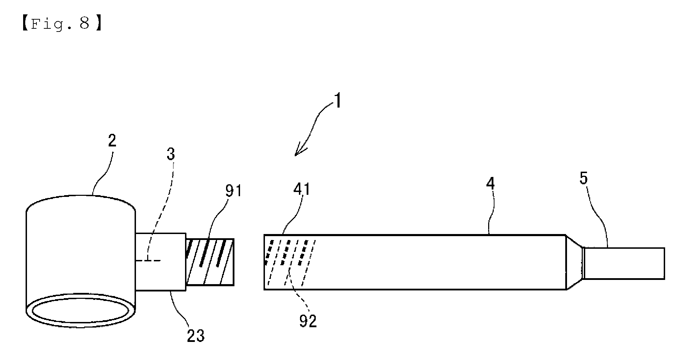

Fig. 8 is an exploded perspective view of a blowout 1 according to a third embodiment of the present invention. This embodiment is different from the first embodiment in the respect that the attaching and detaching means is constituted by an external thread portion (first thread engagement portion) 91 and an internal thread portion (second thread engagement portion) 92. The other component elements of the second embodiment are given the same reference numerals as those of the first embodiment, as long as the former are identical to the latter. Thus, their description is omitted.

The external thread portion 91 is a cylindrical body provided in the one end part 23 of the stretchable body 2 such that the external thread portion 91 is concentric therewith. The external thread portion 91 has an external thread formed in the external circumferential surface. The internal thread portion 92 has an internal thread formed in the internal circumferential surface of the one end part 41 of the pipe body 4. The external thread portion 91 is operable to engage with the internal thread portion 92. The external thread portion 91 engages and couples with the internal thread portion 92 to connect the stretchable body 2 and the pipe body 4, and the external thread portion 91 disengages from the internal thread portion 92 to detach the stretchable body 2 from the pipe body 4.

In the blowout 1 of this embodiment, the external thread portion 91 simply engages with or disengages from the internal thread portion 92, and thereby, the stretchable body 2 can be securely, easily and quickly attached to or detached from the pipe body 4.

Although the embodiments of the present invention have been above described, the present invention is not limited to the embodiments as specific configurations thereof. Without departing from the scope of the present invention, variations or the like in design should be included in the present invention. For example, the attaching and detaching means is not limited to those of the above embodiments, and hence, the attaching and detaching means may have any configuration as long as it connects the one end part 23 of the stretchable body 2 and the one end part 41 of the pipe body 4 so that the stretchable body 2 can be attached to and detached from the pipe body 4. For example, a one-touch band (e.g., a band is tightened by pushing a lever thereof) having a circle/ring shape may be provided in the one end part 23 of the stretchable body 2. Into the one-touch band, the one end part 41 of the pipe body 4 is inserted, and then, the one-touch band is tightened or loosened so that the stretchable body 2 can be attached to and detached from the pipe body 4. Further, the stretchable body 2 may have a scale so that a user can visually check easily how much the stretchable body 2 is stretched.

### [Description of the Symbols]

1 blowout
2 stretchable body
23 one end part
3 wire body
4 pipe body
41 one end part
42 other end part
5 mouthpiece body
6 air regulating body (flow-rate regulating means)
7 tightening band (attaching and detaching means)
8 rubber ring (attaching and detaching means, elastic body)
91 external thread portion (attaching and detaching means, first thread engagement portion)
92 internal thread portion (attaching and detaching means, second thread engagement portion)

## Claims

1. A blowout including: a stretchable body having a tube shape and changeable in shape; a wire body having elasticity and arranged along the longitudinal directions of the stretchable body; a pipe body having a cylinder shape, one end part of the pipe body being connected to one end part of the stretchable body; and a mouthpiece body having a cylinder shape and connected to the other end part of the pipe body, the mouthpiece body being held by a user in the mouth such that air is blown by the user, in which the stretchable body is stretched by blowing air from the mouthpiece body while the stretchable body is curled into a spiral by the elastic force of the wire body when air is not blown from the mouthpiece body,
wherein the one end part of the stretchable body and the one end part of the pipe body are connected by an attaching and detaching means so that the stretchable body is attachable to and detachable from the pipe body.

2. The blowout according to claim 1, wherein:
the attaching and detaching means is a tightening band having a ring shape and flexible in internal shape; and
the one end part of the pipe body is inserted into the one end part of the stretchable body, the tightening band is tightened from above on the one end part of the stretchable body to connect the stretchable body and the pipe body, and the tightening band is loosened to detach the stretchable body from the pipe body.

3. The blowout according to claim 1, wherein:
the attaching and detaching means is an elastic body having flexibility, the elastic body being arranged in the one end part of the stretchable body such that the elastic body extends in the circumferential directions of the stretchable body; and
the one end part of the pipe body is inserted into the one end part of the stretchable body and thereby is pressed by the elastic body to connect the stretchable body and the pipe body, and the pipe body is drawn from the stretchable body to detach the stretchable body from the pipe body.

4. The blowout according to claim 1, wherein:
the attaching and detaching means is constituted by a first thread engagement portion provided in the one end part of the stretchable body and a second thread engagement portion provided in the one end part of the pipe body, the first thread engagement portion being operable to engage with the second thread engagement portion; and
the first thread engagement portion engages with the second thread engagement portion to connect the stretchable body and the pipe body, and the first thread engagement portion disengages from the second thread engagement portion to detach the stretchable body from the pipe body.
